# EUROPÄISCHE PATENTSCHRIFT

(11) **EP 3 488 012 B1**
(45) Veröffentlichungstag und Bekanntmachung des Hinweises auf die Patenterteilung: **19.05.2021**
(21) Anmeldenummer: 17745665.4
(22) Anmeldetag: 14.07.2017
(51) Int. Cl.: C12Q 1/6841, C12Q 1/6853

(54) **DNA-SONDEN FÜR EINE IN-SITU HYBRIDISIERUNG AN CHROMOSOMEN**
DNA PROBES FOR IN SITU HYBRIDIZATION ON CHROMOSOMES
SONDES D'ADN POUR HYBRIDATION IN SITU À DES CHROMOSOMES

(30) Priorität: 25.07.2016 DE 102016113681; 19.12.2016 DE 102016124844
(43) Veröffentlichungstag der Anmeldung: 29.05.2019
(73) Patentinhaber: Invivo Biotech Services GmbH, 16761 Hennigsdorf - Brandenburg (DE)
(72) Erfinder: WEGLÖHNER, Wolfgang, 16761 Hennigsdorf - Brandenburg (DE); SCHINDLER, Sabrina, 16761 Hennigsdorf - Brandenburg (DE)
(74) Vertreter: Boßmeyer, Jens
(86) Internationale Anmeldenummer: PCT/EP2017/067801
(87) Internationale Veröffentlichungsnummer: WO 2018/019610

(56) Entgegenhaltungen:
- DE-A1- 10 240 021
- GISSELSSON DAVID: "Refined characterisation of chromosome aberrations in tumours by multicolour banding and electronic mapping resources", METHODS IN CELL SCIENCE, DORDRECHT [U.A.] : KLUWER, NL , Bd. 23, Nr. 1 1. März 2001 (2001-03-01), Seiten 23-28, XP009500605, ISSN: 1381-5741, DOI: 10.1023/A:1013129212367 Gefunden im Internet: URL:http://epo.summon.serialssolutions.com /2.0.0/link/0/eLvHCXMwlV3PS8MwFA5TELz4G60_ IAdBL5E2adP0KMPNg6c5BU8laRIR3Vba9bD_3pe2dm Ve9BAKbV4oeSHf95rX7yF0TUPJFRU-iXmmSSg1JyLJ FAGqr8PEZJLX8sVvw3A6jifj8GmA_O7Txfzz7udEst 6o-1o8AQN0orXoGGy6bjG58Pz5tdt6aVKXaw0AhkgE WLmh5dOz7wFOdwK6oRZaI8xo___vdoD2WjaJ7xv3H6 KBmR-hnaa-
- SHELAGH BOYLE ET AL: "Fluorescence in situ hybridization with high-complexity repeat-free oligonucleotide probes generated by massively parallel synthesis", CHROMOSOME RESEARCH, KLUWER ACADEMIC PUBLISHERS, DO, Bd. 19, Nr. 7, 18. Oktober 2011 (2011-10-18), Seiten 901-909, XP019974141, ISSN: 1573-6849, DOI: 10.1007/S10577-011-9245-0
- QUNFENG LOU ET AL: "Single-copy gene-based chromosome painting in cucumber and its application for chromosome rearrangement analysis in Cucumis", THE PLANT JOURNAL, Bd. 78, Nr. 1, 12. März 2014 (2014-03-12), Seiten 169-179, XP055413186, GB ISSN: 0960-7412, DOI: 10.1111/tpj.12453
- BOGOMOLOV A G ET AL: "Fluorescence in situ hybridization with DNA probes derived from individual chromosomes and chromosome regions", MOLECULAR BIOLOGY : COVER-TO-COVER TRANSLATION = MOLEKULYARNAYA BIOLOGIYA, ACADEMY OF SCIENCES OF THE USSR, RU , Bd. 48, Nr. 6 1. November 2014 (2014-11-01), Seiten 767-777, XP009500604, ISSN: 0026-8933, DOI: 10.1134/S002689331406003X Gefunden im Internet: URL:http://epo.summon.serialssolutions.com /2.0.0/link/0/eLvHCXMwnV1LS8QwEB5UEL34BldX yEEvYqWmaZoel9XVg3jwhZ5C8yguuqvsQ9h_70wf62 M9iMeWSVomk8wrMx_AvlNGoibPAlKOgYhsHBgb5YGj qL7IpROGAvqPbXF7nlyfi8s54NPQRf_5uM5IFgd1CT siqKSXS8KGR48AlXT0gMcuiRM56Df308QBqe_yVocM iLpKZP46w3dV9Glf_kiJFpqms_qff1yDlcquZK1SEN Zhzvc3YLFE

## Beschreibung

### GEBIET DER ERFINDUNG

Die Erfindung betrifft Verfahren zur Herstellung von Sonden für eine *in-situ-*Hybridisierung von Chromosomen zur Diagnose von Chromosomenaberrationen sowie die Verwendung damit hergestellter DNA-Sonden und Sondengemische.

### HINTERGRUND DER ERFINDUNG

In Tumorzellen sind oft Chromosomenaberrationen zu beobachten. Man kann sie durch G-Bänderung feststellen oder *in-situ*-Hybridisierung (ISH) mit Sonden für bestimmte Genomloci. Bei der Mehrfarben-Fluoreszenz-*in-situ*-Hybridisierung werden mehrere Genombereiche verschiedenfarbig markiert, welche auch entfernt liegen können von den Loci der Krankheiten und Bruchpunkte, so dass man auch komplexe Chromosomenaberrationen einfach diagnostizieren kann. Manche Abberationen treten nur bei bestimmten Tumoren auf, beispielsweise das Philadelphia-Chromosom bei bestimmten Leukämien, andere geben die Art der Behandlung vor wie besonders bei den Tumoren der Brust. Klinisch relevant sind hierbei das Onkogen ERBB2, welches einen Zelloberflächenrezeptor kodiert, und der Bereich CEN17 für die Zentromerregion des Chromosoms 17. Die ISH erlaubt dann eine Aussage über die Aggressivität des Tumors und eine gezieltere Behandlung der Patienten. Auch bei den Non-Hodgkin-Lymphomen lassen sich Untergruppen über genetische Veränderungen unterscheiden.

Klassische Verfahren zur Herstellung von ISH-Sonden verwenden BACKlone (Bacterial Artificial Chromosome), YAC-Klone, Cosmide und Fosmide. Diese enthalten große Bereiche genomischer DNA (bis zu 500 Kilobasen) und damit neben den gesuchten auch repetitive Sequenzen, Pseudogene und paraloge Sequenzen. Diese führen bei der chromogenen oder fluoreszierenden ISH zu unspezifischem Hybridisierungen und Hintergrund, was eine Auswertung erschwert und manchmal unmöglich macht. Besonders in der CISH (Chromogenen-*in-situ*-Hybridisierung) ist ein hoher Hintergrund oft nicht vermeidbar. CISH-Sonden sind in der Regel kleiner als FISH (Fluoreszierende-*in-situ*-Hybridisierungs-) Sonden.

Um Hintergrund durch repetitive Sequenzen zu vermeiden, wird im Fall von BAC-Sonden gerne ein hoher Überschuss an sogenannter Blocking-DNA (z.B. Cot-1 DNA, Salmon Sperm DNA, tRNA, o.a.) zugesetzt. Dadurch lassen sich aber trotzdem Signale von den repetitiven Sequenzen nicht komplett vermeiden. Deshalb versucht man oft auch, die BAC-Klone von repetitiven Sequenzen "zu befreien"; siehe Swennenhuis JF et al, Construction of repeat-free fluorescence in situ hybridization probes, 2011, Nucleic Acids Research, 2012, Vol. 40, No. 3, e20, doi:10.1093/nar/gkr1123; WO 2007/053245, und Stand der Technik in Figur 2. Die BAC-Klone werden zufällig fragmentiert, die Fragmente mit Linkern versehen und in einer PCR amplifiziert. Nach Zusatz eines Überschusses von Cot-1DNA wird die DNA aufgeschmolzen und bei 65°C mit einer Doppelstrang-spezifischen Nuklease (DSN - Duplex specific nuclease) verdaut. Die Schritte Amplifikation und Verdau in Gegenwart von Cot-1DNA werden mehrfach wiederholt, bis man eine Sonde bzw. ein Sondengemisch "frei" von repetitiven humanen Sequenzen erhält. Die Entfernung der repetitiven Sequenzen kann auch durch andere Verfahren erfolgen; siehe Craid JM et al, Removal of repetitive sequences from FISH probes using PCR-assisted affinity chromatography, HUMAN GENETICS (Springer, Berlin, DE) Bd. 100, Seiten 472-476; US 2004029298, WO 2004/083386; WO 01/06014. Diese Verfahren sind aber nicht nur mit erheblichem Aufwand verbunden, sondern auch leider nie vollständig und sicher. Auch kann die Sonden-DNA nicht einfach in Plasmiden gesichert werden.

ISH-Sonden können auch in einer PCR (Polymerasekettenreaktion - polymerase chain reaction) hergestellt werden. Die PCR erfolgt am Genom und gezielt an Bereichen, die frei von repetitiven Sequenzen sind und oft nur einen Teil eines Gens oder einer Domäne kodieren. Repetitive und Alu-Sequenzen sind aber überall im menschlichen Genom und auf allen Chromosomen zu finden. US 8,407,013 B2 offenbart eine computerunterstützte Sequenzanalyse und eine *ab initio* Generierung von Genomsonden durch PCR; siehe Rogan PK et al Sequence-based design of single-copy genomic DNA probes for fluorescence in situ hybridization, Genome Res. (2001) 11(6): 1086-1094. Zudem zeigt Bienko et al., A versatile genome-scale PCR-based pipeline for high-definition DNA FISH, Nature Methods, 2013 Februar; 10(2): 122-124 einen in-silico Aufbau einer Datenbank mit PCR-Primern für nicht-repetitive chromosomale Regionen zur FISH-Detektion. Zum Stand der Technik siehe auch US 6 150 160-A; US 6 828 097 B1; US 7014997 B2; US 2000 3002204 A1; EP 1127163; US 2000 30108943 A1; DE 69032920; US 2000 30194718 A1; US 2000 40161773 A1; WO 2004/04097050; WO 2004/083386; EP 1285093; WO 2014/036525-A1; WO 2000/188089; EP 2069537; EP 1127163; EP 1669902; und Verfahren gemäß DE 10 2010 029 855 A1, sowie Gisselsson, D.,METHODS IN CELL SCIENCE, ORDRECHT [U.A.] : KLUWER, NL,Bd. 23, Nr. 1 1. März 2001, Seiten 23-28; Shelagh Boyle et al.CHROMOSOME RESEARCH, Bd. 19, Nr. 7, 18. Oktober 2011, Seiten 901-909; DE 102 40 021; Qunfeng Lou et al. THE PLANT JOURNAL, Bd. 78, Nr. 1, 12. März 2014, Seiten 169-179; und Bogomolov, A.G. et al. MOLECULAR BIOLOGY: MOLEKULYARNAYA BIOLOGIYA, ACADEMY OF SCIENCES OF THE USSR, RU, Bd. 48, Nr. 6 1. November 2014, Seiten 767-777; und US 2010/240880 A1. Die Herstellung von Sonden frei von repetitiven Sequenzen bleibt aber problematisch, da die Abdeckung derartiger Sonden oft nicht groß ist. Mehrere Megabasen sind bei klinisch relevanten ISH-Sonden aber gefordert.

Die Sonde muss zudem mit radioaktiven, chromogenen oder fluoreszierenden Gruppen markiert werden. Die Markierung kann enzymatisch durch eine Nick- und Translationsreaktion erfolgen, durch Random Priming oder direktes PCR-Labelling mit markierten Nukleotiden und/oder durch eine chemische Kopplung. Die Markierung in der Amplifikationsreaktion ist aber aufwändig, denn für jede Markierung, jeden Fluoreszenzfarbstoff und jede chromogene Gruppe muss die Polymerasekettenreaktion neu etabliert werden. Sie ist auch abhängig von Sequenzlänge, chemischer Struktur der Modifikation, Länge des Linkers zwischen Markierung und Nukleotid, und auch von der Polymerase und der Beschaffenheit des Ausgangsmaterials. Der Stand der Technik repräsentiert somit ein Problem.

### ZUSAMMENFASSUNG DER ERFINDUNG

Das Problem wird gelöst durch das Verfahren gemäß Anspruch 1 und durch Sonden gemäß der Ansprüche 13 und 14. Vorteilhafte Ausführungen des Verfahrens und der Sonden sind den Unteransprüchen zu entnehmen und in den Beispielen beschrieben.

Das Verfahren umfasst die Herstellung direkt oder indirekt markierter Nukleinsäuren, umfassend ein Analysieren von Sequenzen in größeren genomischen Bereichen auf Abschnitte mit spezifischen Sequenzen und Auswählen spezifischer Nukleinsäuresequenzen für bestimmte Orte (Loci); ein Entwerfen und Synthetisieren von *Sense-* und *Antisense*-Primerpaaren für eine Polymerasekettenreaktion an ausgewählten spezifischen Nukleinsäuresequenzen, wobei die synthetisierten Primer jeweils eine Sequenz komplementär zum Strang oder Gegenstrang der nicht spezifischen Nukleinsequenz enthalten und eine nichtkomplementäre einheitliche Linkersequenz, welche unter stringenten Bedingungen nicht mit dem Genom hybridisiert und fakultativ eine Schnittsequenz für eine Restriktionsendonuklease enthalten kann; eine Anzahl erster Polymerasekettenreaktionen mit der Anzahl *Sense-* und *Antisense*-Primerpaaren und nach Vereinigung der Reaktionsprodukte das Erhalten eines ersten Gemisches (Pool A) synthetisierter PCR-Fragmente, die bekannte (nicht-repetitive) Sequenzen enthalten; eine Multiplex-Polymerasekettenreaktion an dem Gemisch (Pool A) synthetisierter PCR-Fragmente unter Verwendung von Primern, welche an den nichtkomplementären Linkersequenzen hybridisieren und Erhalten eines Gemisches (Pool B) mit vervielfältigten sequenzkontrollierten PCR-Fragmenten ohne repetitive Anteile, welche dann einzeln oder im Gemisch geeignet sind für eine chromogene oder fluoreszierende *in-situ*-Hybridisierung (CISH oder FISH) von Chromosomen.

In einer Ausführungsform werden die nach der ersten PolymeraseKettenreaktion im Gemisch vorliegenden synthetisierten Nukleinsäurefragmente größenselektiv analysiert und anschließend aufgereinigt. Weiterhin können vorteilhaft im letzten Amplifikationsschritt modifizierte oder markierte Nukleotide (PCR-Labelling) zugesetzt werden. Werden im letzten Amplifikationsschritt modifizierte Nukleotide zugesetzt, so können dies solche sein, welche eine chemische Kopplung mit einem chromogenen oder fluoreszierenden Gruppe erlauben, bevorzugt Aminoallyl-NTPs.

In einer alternativen Ausführungsform werden die nach der ersten Polymerasekettenreaktion resultierenden Nukleinsäurefragmente in Plasmide kloniert. Der Fachmann erkennt, dass dies unter Restriktion in die Linkersequenz erfolgen kann. Über den Linker können die Fragmente nach Amplifikation der Plasmide in beliebiger Menge generiert werden.

Sondenfragmente können auch einer Reaktion unterworfen werden, welche Nachweisgruppen in die Hybridisierungssonde einbaut oder anhängt. Eingebaute Markierungen können radioaktiv, farbgebend oder fluoreszierend sein. Zu den farbgebenden Gruppen gehören auch Haptene wie Biotin, Avidin, Digoxigenin, denn diese Haptene können in einer Immunreaktion mit einem markierten Antikörper in bekannter Weise sichtbar gemacht werden. Weitere farbgebende Gruppen sind Enzyme, welche eine Farbreaktion katalysieren wie beispielsweise Peroxidasen oder Laktase. Es können auch modifizierten Nukleotide mit einer reaktiven Gruppe wie Allylamin eingesetzt werden, die mit entsprechenden Farbstoffgruppen umgesetzt werden können.

Das offenbarte Verfahren bietet den Vorteil, dass die in Schritt (a) ausgewählten nicht-repetitiven Nukleinsäurensequenzen so gewählt werden können, dass sie in der ersten multiplen Polymerasekettenreaktion mit im wesentlichen gleicher Häufigkeit vervielfältigt werden. Vorzugsweise werden die Sequenzabschnitte so gewählt in Schritt (a), dass nicht-repetitive PCR-Fragmente mit 100 bis 5000 Basenpaare, bevorzugt mit 100 bis 1000 Basenpaaren resultieren. Ganz besonders bevorzugt sind Fragemente mit 400 bis 600 Basenpaaren. Weiterhin liegen vorteilhaft die im Analyseschritt ausgewählten nicht-repetitiven Nukleinsäurensequenzen auf dem zu untersuchenden Genom zueinander benachbart, so dass eine höhere Signalintensität bei der *in-situ*-Hybridisierung resultiert.

Für den Nachweis von Chromosomenaberrationen sind oft mehrere Sonden mit unterschiedlichen Markierungen erforderlich. Offenbart ist daher auch die Herstellung einer Mehrzahl Sonden mit unterschiedlichen Markierungen. Es ist vorteilhaft, wenn die im ersten Schritt ausgewählten spezifischen Nukleotidsequenzen zu einem Bruchbereich im Chromosom benachbart sind. Besonders vorteilhaft und praktisch für die Diagnostik ist, wenn die im Analyseschritt ausgewählten spezifischen Sequenzen einen Bruchbereich flankieren und unterschiedlich markiert sind, da so unmittelbar eine Chromosomenaberrationen diagnostiziert werden kann. Die verschiedenen Markierungen der Sonden können auch so für benachbarte Sequenzen gewählt werden, dass die Farbmarkierungen zunächst eine Mischfarbe ergeben und bei einer Aberration ein Farbwechsel zu beobachten ist bzw. zwei unterschiedliche Farbsignale. Auch der umgekehrte Vorgang kann erfolgen, also zwei unterschiedliche Farbsignale im Fall einer Aberration ein Mischsignal bzw. ein Fusionssignal produzieren. In anderen Fällen können auch Sequenzen gewählt werden, die aus einem Bereich sind oder diesen flankieren, der bei einer Aberration vervielfältigt wird, gegebenenfalls im Rahmen einer balancierten, unbalancierten und reziproken Translokation.

Die Markierung der Sonden ist bevorzugt ausgewählt aus der Gruppe: farbgebende Molekülen, Polymethin-Farbstoffe, Thiazol- und Oxazolfarbstoffe, Hoechst 33342 (2'-(4-Ethoxyphenyl)-5-(4-methyl-1-piperazinyl)-2,5'-bi-1H-benzimidazol trihydrochlorid), 4',6-Diamidin-2-phenylindol, Alexa 405, Alexa 488, Alexa 594, Alexa 633; Texas Red, Rhodamin; sulfonierte und nicht-sulfonierte Cyanin-Farbstoffe, Cy2, Cy3, Cy5, Cy7; fluoreszierenden Moleküle, Fluorescein, 5,6-Carboxfluorescein, FITC (Fluoresceiniso thiocyanat), GFP (grünfluoreszierendes Protein); chemilumineszierende Mokeküle, Acridinium; ATTO®-Fluoreszenzfarbstoffe (Atto-Tec, Siegen, DE), PromoFluor®-Farbstoffe (PromoCell GmbH, Heidelberg, DE), MoBiTec®-Farbstoffe (MoBiTec GmbH, Goettingen, DE), DY®-Farbstoffe (DYOMICS GmbH, Jena, DE) Quantum Dots; Haptene, Digoxigenin, Biotin, 2,4-Dinitrophenol, Avidin; Enzyme für eine farbgebende Reaktion, Peroxidase, Meerrettichperoxidase, alkalische Phosphatase.

Eine Ausführungsform betrifft die Bereitstellung einer markierten Sonde für eine *in-situ*-Hybridisierung zum Nachweis einer Chromosomenaberration, bestehend aus einer Mehrzahl PCR-Fragmente, deren Sequenzen keine Wiederholungen, Pseudogene und paralogen Gene enthalten und die auf dem Humangenom benachbart sind. Eine weitere Ausführungsform betrifft ein Sondengemisch beziehungsweise ein Nachweisbesteck für eine bestimmte Chromosomenaberration, das mehrere unterschiedlich markierte Sonden enthält, welche die jeweiligen Bruchpunktbereiche flankieren.

Es werden weitere Vorteile, Ausführungsformen und Vorteile der Erfindung in Beispielen und mit Bezug auf die anliegenden Abbildungen beschrieben.

### KURZBESCHREIBUNG DER ABBILDUNGEN

Es zeigt:
- Fig. 1: eine zeichnerische Darstellung der Schritte zur Herstellung sequenzkontrollierter PCR-Sonden für die *in-situ*-Hybridisierung (ISH) von Chromosomen;

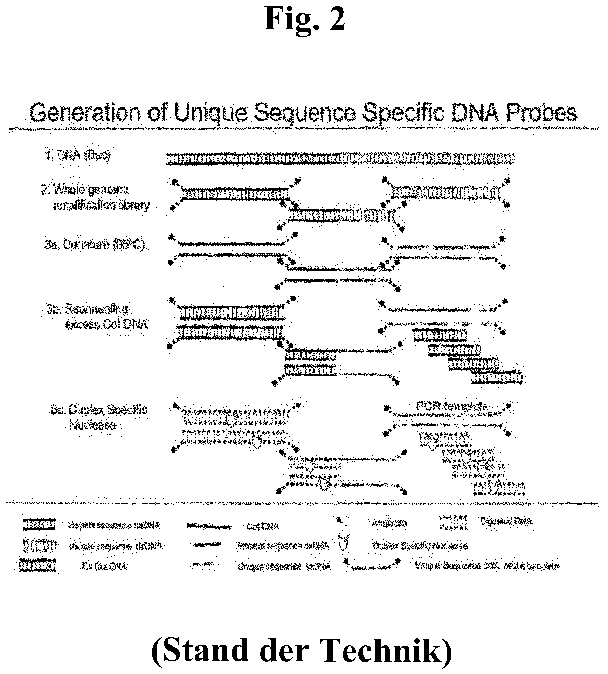

- Fig. 2: eine Schemazeichnung der Schritte zur Gewinnung von Repeat-reduzierter ISH-Sonden nach dem Stand der Technik;
- Fig. 3: Fluoreszenz-Mikroskopaufnahmen von Proben nach Kontrollfärbung des Zellkerns mit DAPI (4',6-Diaminophenolindol) sowie verschiedenen *in-situ-*Hybridisierungen, wobei die sequenzkontrollierte ISH-Sonde in der PCRReaktion ("One-Step") markiert wurde: linke Spalte: Kontrollfärbung des Zellkerns mit DAPI; rechte Spalte: *in-situ*-Hybridisierung mit Sonden für die Gene HER2, MDM2, MET und FGFR1 bzw. das Zentromer;
- Fig. 4: Fluoroszenzmikroskopaufnahmen von Proben nach Kontrollfärbung des Zellkerns mit DAPI (4',6-Diaminophenolindol) und verschiedenen *in-situ-*Hybridisierungen, wobei die sequenzkontrollierte ISH-Sonde durch Nicktranslation markiert wurde: linke Spalte: Kontrollfärbung des Zellkerns mit DAPI; rechte Spalte: *in-situ*-Hybridisierung mit Sonden für die Gene HER2, MDM2, MET und FGFR1 bzw. das Zentromer.

### DETAILLIERTE BESCHREIBUNG DER ERFINDUNG

Die Generierung sequenzkontrollierter PCR-Sonden kann von genomischer DNA erfolgen. Die vollständige Sequenz des Humangenoms ist bekannt und kann Datenbanken entnommen werden. Sie ist der Ausgangspunkt für das Design der FISH/CISH-Sonden. Es kann auch mit der Sequenz von BAC-Klonen oder anderen DNA-Trägern (Plasmide, Cosmide, Fosmide, YACs) begonnen werden, sollten die Träger und deren Sequenzen zur Verfügung stehen. Alles was nachstehend an genomischer DNA beschrieben wird, kann auch mit den anderen Trägern erfolgen.

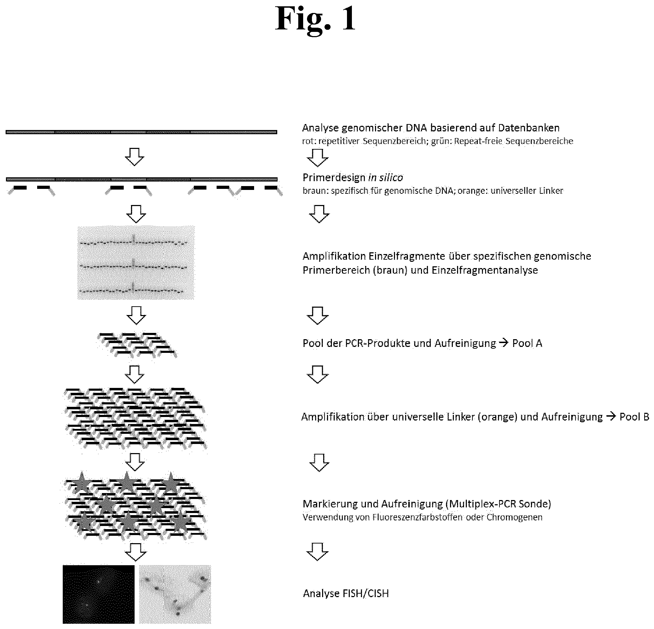

Siehe Figur 1. In einem ersten Schritt (a) werden die genomische Sequenz und die genetische Zusammenstellung der Region anhand der Sequenzen in den Datenbanken mittels Computeranalyse untersucht. Gesucht und identifiziert werden in der genomischen Sequenz hierbei alle Bereiche mit einfachen Repeats, Alu-Sequenzen und komplexen Wiederholungen sowie alle Pseudogene und paralogen Abschnitte. Diese Sequenzen sind für die ISH-Sonde ungeeignet. Öffentliche Genomdatenbanken stellen bereit das NCBI (National Center for Biotechnology Information, Bethesda, Maryland) oder sind ENSEMBL (gepflegt vom European Bioinformatics Institute (EBI) und dem European Molecular Biology Laboratory (EMBL), Heidelberg, DE) oder sind zugänglich über den UCSC-Genome Browser (University of California, Santa Cruz, US). Eventuelle unspezifische Sequenzbereiche können unter Verwendung gängiger Analyseprogramme weiter eingegrenzt und identifiziert werden.

In die Hybridisierungssonde werden nur Sequenzen aufgenommen, die spezifisch für einen Lokus sind und einmal vorkommen. Ausgeschlossen werden Sequenzen, die bei einer Chromosomenhybridisierung auch an anderen Loci hybridisieren und somit unspezifisch zum Hintergrund beitragen (Schritt a). Für die so kontrollierten genomischen Sequenzen, welche nur einmal im Chromosom vorkommen, wird dann (b) eine Bibliothek von *Sense-* und *Antisense*-Primern erstellt. Die Primer werden synthetisiert jeweils mit einer spezifischen Primersequenz und einem uniformen Linker. *Sense-* und *Antisense-*Primer werden auf Basis der bekannten Genomsequenz so geplant und gewählt, dass bei der PCR und der nachfolgenden Amplifikation im Wesentlichen gleich lange DNA-Fragmente resultieren. Es werden für jeden Genombereich typisch 200 Primer entworfen und synthetisiert, so dass nach der PCR der Lokus auf dem Genom abschnittsweise von 100 DNA Fragmenten abgedeckt wird. In einer ersten Amplifikation werden mittels der Primer die spezifischen Fragmente in individuellen PCR-Reaktionen generiert, die resultierenden PCR Fragmente auf Reinheit und Größe überprüft und in einem Schritt (d) miteinander vereinigt bzw. gepoolt (Pool A). Nach einer Aufreinigung des Pools (Pool A) werden 10 ng gepoolte PCR-DNA als Matrize für eine zweite Amplifikation verwendet - (Schritt e) - wobei in dieser Amplifikation die Linker als *Sense-* und *Antisense*-Primer dienen. Da alle Fragmente des A-Pools gleiche Linker besitzen und eine geplante ähnliche Größe, werden alle PCR-Fragmente aus Pool A in dieser Reaktion gleichermaßen vervielfältigt (Multiplex-PCR). Es resultiert ein gemischter Pool PCR-Fragmente (Pool B).

Werden in dieser letzten Reaktion markierte Nukleotide zugesetzt (One-Step-PCR-Labelling), resultiert ein Pool B mit markierten PCR-Fragmenten ähnlicher Größe. Nach Aufreinigung können diese in der *in-situ*-Hybridisierung eingesetzt werden. Alternativ kann der Pool B mit den gemischten PCR-Fragmenten nach der zweiten Vervielfältigung auch durch Nick-Translation markiert werden.

Um einen Genlokus robust in der FISH/CISH detektieren zu können, müssen in der Regel genomische Bereiche von 100 bis 1000 Kilobasen für das Sondendesign untersucht werden. Es werden also 1 bis 10 Multiplex-PCR-Sonden hergestellt, die in Kombination die eigentliche Sonde ergeben. Die so erzeugte Sonde enthält bereits von Beginn der Herstellung an keine repetitiven Elemente, Pseudogene oder Paraloge. Die Sonde kann in beliebiger Menge hergestellt werden. Da die einzelnen PCR-Fragmente im Wesentlichen ähnlich groß sind, beziehungsweise ihre Länge vorgegeben ist, stellt der direkte Einbau von markierten Nukleotiden keine großen Anforderungen mehr. In der üblichen Multiplex-PCR oder Linker-PCR können eingesetzt werden: (a) nicht chemisch veränderte bzw. unveränderte dNTPs, wenn die nachfolgende Markierung durch klassische enzymatische Verfahren wie beispielsweise Nick-Translation erfolgt, (b) mit Fluoreszenzfarbstoffen (beispielswiese Atto488 oder Cy3) oder Haptenen (zum Beispiel Digoxigenin-, Dinitrophenol- oder Biotin-) markierte dNTPs, wobei die Markierung direkt in der Linker-PCR erfolgen kann. Für die Zugabe der Fluoreszenzfarbstoff- bzw. Haptenmarkierten dNTPs muss man das Verhältnis bestimmen, in dem diese zugesetzt werden. Dieses wird je nach Markierung und dNTP (dUTP : fluoreszenzmarkiertedNTP) zwischen 1:1 und 1:20 liegen; oder (c) chemisch reaktive dNTPs wie bspw. Aminoallyl-dNTP. Die Markierung kann auch nach der Synthese durch chemische Kopplung mit aminreaktiven NHS-Ester-aktivierten (NHS = N-hydroxysuccinimid) Farbstoff oder Haptenderivaten erfolgen. Die Zugabe reaktiver Aminoallyl-Nukleotiden bzw. Aminoallyl-dNTPs erfolgt, je nach Sondentyp (Gensonde oder Zentromersonde) im Verhältnis zwischen 5:1 und 1:5. Die DNA-Sonde wird dann post Synthese durch Umsetzen mit aminreaktivem Farbstoff bzw. Fluoreszenzfarbstoff markiert. Erfolgt die Markierung der DNA-Sonde nicht direkt mit Fluoreszenzfarbstoffen oder Haptenen, muss die Markierung im Anschluss an die Linkeramplifikation erfolgen.

### BEISPIELE

### Beispiel 1 Herstellung einer Lokussonde für das Gen HER2

### 1. Design der HER2-Lokussonde in silico

Für das Design der HER2(ERBB2)-Gensonde wurde auf Chromosom 17 der Genombereich mit dem HER2-Gen und große Flanken 5' und 3' dazu ausgewählt. Die HER2-Gensonde deckte auf Chromosom 17 ab den Bereich von 39.395.605 bis 39.799.506. Die genomische Sequenz wurde in den Datenbanken Ensembl (www.ensembl.org) und NCBI (https://www.ncbi.nlm.nih.gov/) analysiert und vier geeignete Abschnitte für die Sonde identifiziert. Der erste Genomabschnitt war Chromosom 17: 39. 395.605 - 39.569. 361. Die nachstehende Darstellung betrifft nur diesen Abschnitt; die drei anderen genomischen Abschnitte am HER2-Lokus wurden analog abgearbeitet.

Im Genomabschnitt 39.395.605 - 39.569.361 wurden alle repetitiven Elemente, Alu-Sequenzen, Pseudogene und paraloge Sequenzabschnitten mit gängigen Analyseprogrammen identifiziert. Repetitive Elemente, Pseudogene und paraloge Sequenzen wurden ausgeschlossen. Die für den ersten Genomabschnitt spezifischen Sequenzen wurden in Abschnitte mit 250 bis 800 Basenpaaren unterteilt. Teilsequenzen mit weniger als 250 Basenpaaren wurden nicht berücksichtigt. Nach dieser Analyse enthielt die genomische Zielsequenz in den vier gewählten genomischen Abschnitten jeweils nur noch 40 bis 60 Prozent der Ausgangssequenz - circa die Hälfte der genomischen Ausgangssequenz wurde somit als unspezifisch verworfen.

### 2. Primerdesign in silico

Zu den spezifischen Sequenzabschnitten wurden *Sense-* und *Antisense-Primer* geplant und synthetisiert für Genomfragmente mit 250 bis 800 Basenpaaren. Die Primer waren einerseits komplementär zur Genomsequenz, die amplifiziert werden sollte, andererseits enthielten sie auch eine universelle Linkersequenz mit einer Schnittstelle für eine *Restriktionsendonuklease. Sense-* und *Antisense*-Primer waren so geplant, dass in der PCR am Genom die resultierenden Produkte ähnlich lang wurden. Angestrebt wurde eine Fragmentlänge von ca. 500 Basenpaaren. Davon wurde nur abgewichen, wenn es die Spezifität bzw. die Funktionalität des Primerpaares es verlangte. Es wurden jeweils ca. 200 Primer für jeden der vier genomischen Abschnitte entworfen und synthetisiert. Tabelle 1 im Anschluss an die Beschreibung enthält eine repräsentative Liste der so ermittelten *Sense-* und *Antisense-Primer* für den Genomabschnitt 39.395.605 - 39.569.361 auf Chromosom 17.

### 3. Amplifikation der Einzelfragmente

Die Amplifikation der Genomfragmente erfolgte in 50 µl Ansätzen für jedes *Sense-* und *Antisense*-Primerpaar. Die einzelnen PCR-Reaktionen erfolgten im Hochdurchsatzverfahren (96-Well) mit 55°C Anlagerungstemperatur (Primer-Annealing) und jeweils 15 Sekunden Strangverlängerung (Elongation) über 35 Zyklen. Die erhaltenen PCR-Fragmente wurden auf dem Agarosegel auf Reinheit und Größe überprüft. Es wurden nur PCR-Produkte mit spezifischer Bande der erwarteten Größe verwendet, so dass deren Sequenz effektiv kontrolliert und bekannt war. Die Ausbeute an korrekten PCR-Produkten betrug über 90%.

### 4. Schneiden der Einzelfragmente mit Restriktionsenzymen und Klonierung

Die PCR-Produkte wurden in der Regel blunt ohne Schneiden in ein Trägerplasmid kloniert und so gesichert. Einige PCR-Produkte wurden auch nach Schneiden in ein Plasmid kloniert. Die vereinzelt vorgenommene Überprüfung der Sequenzrichtigkeit war unproblematisch und erfolgte in bekannter Weise.

### 5. Poolen und Aufarbeitung der Einzelfragmente

Die ca. 100 PCR-Produkte wurden gepoolt und aufgearbeitet, um Primer, Proteine, freie Nukleotide und Salze zu entfernen. Es folgte eine photometrische Messung und optische Analyse des Gemisches (Pool A) durch Agarose-Gelelektrophorese.

### 6. Amplifikation des Pools über universelle Linker (Multiplex-PCR) und Markierung

Die zweite Amplifikation über die universellen Linker erfolgte mit einer Hochleistungs-Taq-Polymerase für große Ausbeuten, direkt mit dem Gemisch der Einzelfragmente (Pool A) als Template (Multiplex-PCR). In dieser Multiplex-PCR/Linker-PCR wurden mit Fluoreszenzfarbstoffen (z.B. Atto488 oder Cy3) markierte dNTPs eingesetzt. Die Zugabe der fluoreszenzmarkierten dNTPs erfolgte in einem getesten Verhältnis, abhängig vom markierten dNTP, und für die unterschiedlichen Sonden unterschiedlich (Gensonde oder Zentromersonde). Das Verhältnis betrug über die verschiedenen Sonden und je nach Markierung zwischen 5:1 und 1:5.

### 7. Aufreinigung der markierten DNA-Sonde

Nach Markierungs- und Amplifikationsreaktion folgte eine letzte Aufreinigung der markierten Multiplex-PCR-Sonden durch Präzipitation und Chromatographie, wobei freie dNTPs, markierte Nukleotide, *Sense-* und Anti-sense-Primer sowie Enzym entfernt wurden. Es folgte eine photometrische Messung und Bestimmung der Einbaurate an Fluoreszenz in den FISH-Sonden sowie eine finale Überprüfung der Sonde durch Agarose-Gelelektrophorese.

### 8. Zusätzliche Fragmentierung von DNA-Sonden

Optional wurden die DNA-Sonden noch nachfragmentiert, wenn sie wegen ihrer Länge zuviel Hintergrund bei der ISH produzierten. Allgemein ist eine Länge von 200 bis 300 Basenpaaren für die ISH günstig. Die Nachfragmentierung erfolgte physikalisch, kann aber auch enzymatisch oder chemisch erfolgen. Die Fragmentgrößenverteilung wurde durch Agarosegel-Elektrophorese analysiert.

### 9. Fertigstellung der DNA-Sonde

Die HER2-Lokussonde umfasste vier Abschnitte. Die vorstehenden Schritte 1 bis 8 betrafen einen ersten Abschnitt. Die finale DNA-Sonde bestand aus vier getrennten Präparationen (4 Multiplex-PCR-Sonden). Alternativ können diese vier einzelnen Präparationen auch nach der universellen Linker-Amplifikation vereinigt und zusammen markiert und aufgereinigt werden, was aber weniger Kontrolle erlaubt. Je nach Lokus, Sondenart (Amplifikationssonde, Break-Apart-Sonde, Fusionssonde) und Vorgaben der Anwender wird ein DNA-Sondengemisch aus 1 bis 10 Präparationen (Multiplex-PCR-Sonden) bestehen.

### Beispiel 2 In-situ-Hybridisierungen von Chromosomen mit markierten PCR-Sonden

Variante 1: Die ISH mit DNA-Sonden kann mit Standardverfahren an Geweben oder Zellen erfolgen, beispielsweise entsprechend den Empfehlungen des Arbeitskreises Laboratorium der DGHO (Deutschen Gesellschaft für Hämatologie und Onkologie). Die *in-situ* Hybrisierung erfolgt hierbei an Interphasezellen von Zellkulturen oder Geweben. Die Ziel-DNA ist jeweils die nukleäre DNA von Interphasenzellkernen, welche auf einem Objektträger fixiert sind. Die Sonde ist dabei wie in Beispiel 1 beschrieben hergestellt und markiert. Typischerweise werden die Zellkerne mit dem Fluorochrom DAPI (4,6-diamidino-2-Phenylindol) gegengefärbt. FISH kann prinzipiell an folgenden Materialien erfolgen: peripheres Blut (PB), Knochenmark (KM), Paraffinschnitte, Tumorgewebe, Zytospinpräparate, Fruchtwasser, Methanol-Eisessig fixierte Zellen/Metaphasen etc. Andere Patientenproben wie Blut oder Knochenmark werden nach Ficoll-Separation mit Methanol/Essigsäure fixiert (Verhältnis 3:1) und bis zur Hybridisierung bei -20°C eingefroren. Bei Fruchtwasser oder Paraffinschnitten sind besondere Vorbehandlungen notwendig.

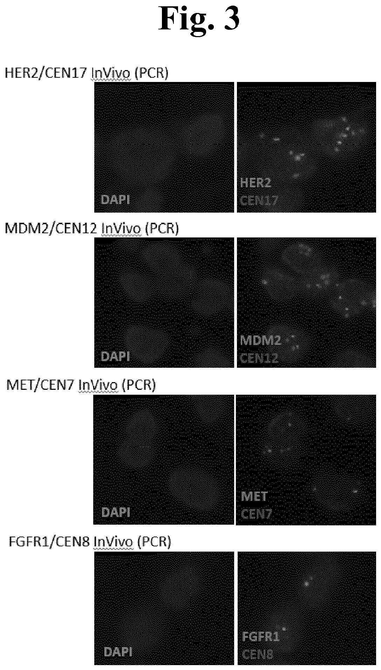

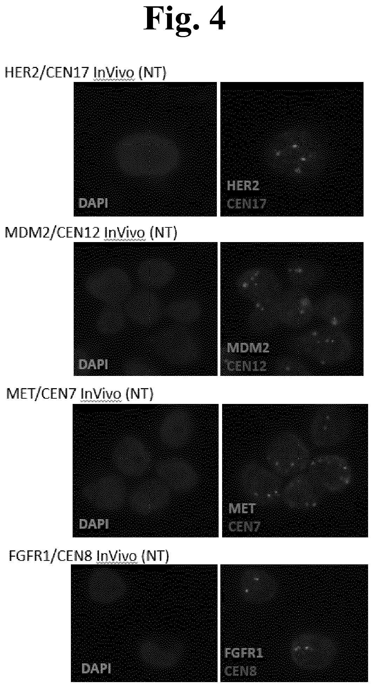

Variante 2: In den Beispielen (siehe Figuren 3 und 4) wurden die in Paraffin gebetteten Zellen von Zelllinien (Zellkulturen) nach einem Standardverfahren am Mikrotom geschnitten und auf einen gereinigten Objektträger gezogen. Die anschließende Behandlung der in Paraffin gebetteten Zellen erfolgte ebenfalls nach Standardverfahren. Die Entparaffinierung wurde mit einer Xylol- und einer Alkoholreihe gewährleistet. Eine Vorbehandlung erfolgte bei 95°C für wenige Minuten in Citrat-Vorbehandlungspuffer. Es folgte ein partieller Verdau der Zellen mit Pepsinlösung, um die Zellkerne möglichst von Zytoplasma zu befreien und zugänglich zu machen. Nach einer Entwässerung in einer Alkoholreihe wurde ein Sonden-Hybridisierung-Gemisch auf die Zellen pipettiert und mit einem Deckglas und Dichtmittel versiegelt. Die Zellkerne und die Hybridisierungsprobe wurden bei 75°C für 10 Minuten denaturiert, langsam auf 37°C abgekühlt und in einer feuchten Kammer bei 37°C über Nacht hybridisiert (16 h). Nach Entfernen des Deckglases folgten zwei Waschungen bei 37°C mit Waschlösung. Die Hybridisierungssignale wurden am Fluoreszenzmikroskop mit entsprechenden Filtern ausgewertet und dokumentiert. Figuren 3 und 4 zeigen die Ergebnisse für unterschiedlich markierte Sonden (durch Nicktranslation bzw. One-Step-PCR-Labelling). In beiden Fällen waren die Hybridisierungssignal klar erkennbar und die Hintergrundhybridisierung war vernachlässigbar.

**TABELLE1**

| *Sense- und Antisense-Primer für den Genomabschnitt 39.395.605* - *39.569.361 auf Chromosom 17.* | | | |
|---|---|---|---|
| **SEQ ID:** | **Name** | **Primersequenz 5' nach 3'** | **Amplifikat in Basenpaaren** |
| SEQ ID NO: 001 | D2_s_254 | TGTAAAACGACGGCCAGTCTTAGTTTCACCAGTACTCAATCCC | |
| SEQ ID NO: 002 | D2_as_254 | CAGGAAACAGCTATGACCCTCAGTCTCAGAATACACCGTTAAG | 522 |
| SEQ ID NO: 003 | D2_s_255 | TGTAAAACGACGGCCAGTCTAGGGTTACAGGTTGCACATAATA | |
| SEQ ID NO: 004 | D2_as_255 | CAGGAAACAGCTATGACCGCCTTCATTTTAAGTGCCATAACTG | 409 |
| SEQ ID NO: 005 | D2_s_256 | TGTAAAACGACGGCCAGTTGGCCTCCAGTTACAACATATAAAA | |
| SEQ ID NO: 006 | D2_as_256 | CAGGAAACAGCTATGACCCTCTGTAGGGGTGGCATACTATAAT | 723 |
| SEQ ID NO: 007 | D2_s_257 | TGTAAAACGACGGCCAGTCCCGACTCAAATCTTCAGTATCTG | |
| SEQ ID NO: 008 | D2_as_257 | CAGGAAACAGCTATGACCAGGGTGTGAAAACTTTTAAGGGTT | 425 |
| SEQ ID NO: 009 | D2_s_258 | TGTAAAACGACGGCCAGTAAAGACCAGGTGACATTTACTCTTC | |
| SEQ ID NO: 010 | D2_as_258 | CAGGAAACAGCTATGACCGAGTTTGAGACCTTGGTGAAACTAG | 456 |
| SEQ ID NO: 011 | D2_s_259 | TGTAAAACGACGGCCAGTAACAAACGCGTCATGAAGGTAG | |
| SEQ ID NO: 012 | D2_as_259 | CAGGAAACAGCTATGACCAAGAGCAGGTTTGAGGTGTGAG | 405 |
| SEQ ID NO: 013 | D2_s_260 | TGTAAAACGACGGCCAGTCAGTGCATTACATTACGGGGTG | |
| SEQ ID NO: 014 | D2_as_260 | CAGGAAACAGCTATGACCAAGTCAGTCGCTAAAACCACTAATT | 744 |
| SEQ ID NO: 015 | D2_s_261 | TGTAAAACGACGGCCAGTTGGCCTACAGAGTAATAGAATGAAG | |
| SEQ ID NO: 016 | D2_as_261 | CAGGAAACAGCTATGACCCCCCATACATTTCTATCCTTGAGT | 503 |
| SEQ ID NO: 017 | D2_s_262 | TGTAAAACGACGGCCAGTTGAGAATTCATGATATTGCTGCACT | |
| SEQ ID NO: 018 | D2_as_262 | CAGGAAACAGCTATGACCGATAAGCCCCTGAATTGTCGATG | 534 |
| SEQ ID NO: 019 | D2_s_263 | TGTAAAACGACGGCCAGTTTCTTTGATCTGGGATGAAGACAG | |
| SEQ ID NO: 020 | D2_as_263 | CAGGAAACAGCTATGACCAGGTTGGAGTCAGCATAGAGTTG | 526 |
| SEQ ID NO: 021 | D2_s_264 | TGTAAAACGACGGCCAGTATATCAAAGTAAGGCCGCAGAATTT | |
| SEQ ID NO: 022 | D2_as_264 | CAGGAAACAGCTATGACCAAACATGTTTTCAGATGCAGTAAGG | 524 |
| SEQ ID NO: 023 | D2_s_265 | TGTAAAACGACGGCCAGTGATTACAAGTCAATACCTCCACAGA | |
| SEQ ID NO: 024 | D2_as_265 | CAGGAAACAGCTATGACCATGTATATGAAGGGCTGGGCAG | 504 |
| SEQ ID NO: 025 | D2_s_266 | TGTAAAACGACGGCCAGTAGTATTTTAACTTTCTTTCTGGCACA | |
| SEQ ID NO: 026 | D2_as_266 | CAGGAAACAGCTATGACCGACAAGTAACACAATCTTATCTGCA | 356 |
| SEQ ID NO: 027 | D2_s_267 | TGTAAAACGACGGCCAGTAGCTGGGACTATTCTGATAAGATTT | |
| SEQ ID NO: 028 | D2_as_267 | CAGGAAACAGCTATGACCTGTCATAGATAAACTGAAGCATGGG | 530 |
| SEQ ID NO: 029 | D2_s_268 | TGTAAAACGACGGCCAGTGTTCTGAGAGGATGACATGGAAG | |

| **SEQ ID:** | **Name** | **Primersequenz 5' nach 3'** | **Amplifikat in Basenpaaren** |
|---|---|---|---|
| SEQ ID NO: 030 | D2_as_268 | CAGGAAACAGCTATGACCAATCAGAAGGTTCATCAAGTTCCAA | 503 |
| SEQ ID NO: 031 | D2_s_269 | TGTAAAACGACGGCCAGTCTGGTATACTGACTGTGAGAGGAAG | |
| SEQ ID NO: 032 | D2_as_269 | CAGGAAACAGCTATGACCAAGTTTAAGGGCAATAACCAAGCC | 500 |
| SEQ ID NO: 033 | D2_s_270 | TGTAAAACGACGGCCAGTCCACTTTGGCTGCTTTCATCAAA | |
| SEQ ID NO: 034 | D2_as_270 | CAGGAAACAGCTATGACCAAGAAGTCATCAAGATTACCACCTG | 602 |
| SEQ ID NO: 035 | D2_s_271 | TGTAAAACGACGGCCAGTTTTCTAAAGGGCTGCTTCCATAAA | |
| SEQ ID NO: 036 | D2_as_271 | CAGGAAACAGCTATGACCTTGTGTGATTCATAAGAGGTTGACA | 720 |
| SEQ ID NO: 037 | D2_s_272 | TGTAAAACGACGGCCAGTACATCTCTAAATGGGACTCAGATGA | |
| SEQ ID NO: 038 | D2_as_272 | CAGGAAACAGCTATGACCCTTTTAGATTCTCCTGGGCTTCTC | 363 |
| SEQ ID NO: 039 | D2_s_273 | TGTAAAACGACGGCCAGTGACTGAAATTCTCTCTCCCTCCTT | |
| SEQ ID NO: 040 | D2_as_273 | CAGGAAACAGCTATGACCAGTAGTTGGAGTAATAACATAGAGCA | 614 |
| SEQ ID NO: 041 | D2_s_274 | TGTAAAACGACGGCCAGTATAGTTCTTTTGACACAGCTTCCAA | |
| SEQ ID NO: 042 | D2_as_274 | CAGGAAACAGCTATGACCTGTTAGCTAGGTTAGGAAATCACTA | 411 |
| SEQ ID NO: 043 | D2_s_275 | TGTAAAACGACGGCCAGTGCAGTCTTGAAGAAACTTATCTGAC | |
| SEQ ID NO: 044 | D2_as_275 | CAGGAAACAGCTATGACCATATCAAGTCAGAATCCAACCCTTC | 646 |
| SEQ ID NO: 045 | D2_s_276 | TGTAAAACGACGGCCAGTGTACAAACTCTCAAAAGCTCACTCT | |
| SEQ ID NO: 046 | D2_as_276 | CAGGAAACAGCTATGACCCTATCCCCAGTTTCTCGATCTTTG | 521 |
| SEQ ID NO: 047 | D2_s_277 | TGTAAAACGACGGCCAGTCATAATGGTTCTGTCTTGTAATTGGG | |
| SEQ ID NO: 048 | D2_as_277 | CAGGAAACAGCTATGACCATGTTCTTCTCATCTACTCTCCAGC | 443 |
| SEQ ID NO: 049 | D2_s_278 | TGTAAAACGACGGCCAGTTGACTGGCTTCACTACTATTAAAGC | |
| SEQ ID NO: 050 | D2_as_278 | CAGGAAACAGCTATGACCTTGAGGAAAATGGTTAAAGCTGATT | 606 |
| SEQ ID NO: 051 | D2_s_279 | TGTAAAACGACGGCCAGTCAACACTTGTGGGTATACAGCA | |
| SEQ ID NO: 052 | D2_as_279 | CAGGAAACAGCTATGACCTATTTTGCTGCTACTCGTTTCTTTT | 431 |
| SEQ ID NO: 053 | D2_s_280 | TGTAAAACGACGGCCAGTGATAGAGTAGGGTGAAAAGCTATGC | |
| SEQ ID NO: 054 | D2_as_280 | CAGGAAACAGCTATGACCTGCTTTGACTTTAAATTTCTCACCC | 419 |
| SEQ ID NO: 055 | D2_s_281 | TGTAAAACGACGGCCAGTCTTGTTAAAGCCTTTGGATGATGTC | |
| SEQ ID NO: 056 | D2_as_281 | CAGGAAACAGCTATGACCCAGCTAACCCTAATCAGTGAACTTT | 658 |
| SEQ ID NO: 057 | D2_s_282 | TGTAAAACGACGGCCAGTTCCCCAGGTATCTTATATTACCCTT | |
| SEQ ID NO: 058 | D2_as_282 | CAGGAAACAGCTATGACCTATATGGATTTTGCATGGATGGACA | 522 |
| SEQ ID NO: 059 | D2_s_283 | TGTAAAACGACGGCCAGTCTCCTAAAGTTGAAACCACCATGTA | |
| SEQ ID NO: 060 | D2_as_283 | CAGGAAACAGCTATGACCTAACTTGTGTGTCATCTTGCCATTA | 421 |
| SEQ ID NO: 061 | D2_s_284 | TGTAAAACGACGGCCAGTAGAACTATGCCCAAAATGCTAAGG | |
| SEQ ID NO: 062 | D2_as_284 | CAGGAAACAGCTATGACCTTTAGATTGTCACTATACCCTCCTT | 451 |
| SEQ ID NO: 063 | D2_s_285 | TGTAAAACGACGGCCAGTCTCCTTTCCCCTACAGTCACTTA | |
| SEQ ID NO: 064 | D2_as_285 | CAGGAAACAGCTATGACCATCTGGAGATTTTAATTTGCGAGCT | 506 |
| SEQ ID NO: 065 | D2_s_286 | TGTAAAACGACGGCCAGTGTGAAACGGAGGCAAGAAAGGG | |
| SEQ ID NO: 066 | D2_as_286 | CAGGAAACAGCTATGACCGCGAGGGAAGAAATGAGAGTTC | 319 |
| SEQ ID NO: 067 | D2_s_287 | TGTAAAACGACGGCCAGTCTTCTTCAGGTCAGGGGAAAGG | |
| SEQ ID NO: 068 | D2_as_287 | CAGGAAACAGCTATGACCATAGTCATCAGTCTCCTCATTCGAA | 507 |
| SEQ ID NO: 069 | D2_s_288 | TGTAAAACGACGGCCAGTACAAAGACCGGAGTAAAAGTCATC | |
| SEQ ID NO: 070 | D2_as_288 | CAGGAAACAGCTATGACCCAGACAATGTTAACACGCTGAATG | 545 |
| SEQ ID NO: 071 | D2_s_289 | TGTAAAACGACGGCCAGTCAGAATTCGAGAAGTGAAGAGTACA | |
| SEQ ID NO: 072 | D2_as_289 | CAGGAAACAGCTATGACCACAAAATTTCAAACTAGCATGCCTT | 589 |
| SEQ ID NO: 073 | D2_s_290 | TGTAAAACGACGGCCAGTAACTTAAAATGATCTGCCCCATAGA | |
| SEQ ID NO: 074 | D2_as_290 | TGTAAAACGACGGCCAGTAACTTAAAATGATCTGCCCCATAGA | 451 |
| SEQ ID NO: 075 | D2_s_291 | TGTAAAACGACGGCCAGTAACTTAAAATGATCTGCCCCATAGA | |
| SEQ ID NO: 076 | D2_as_291 | CAGGAAACAGCTATGACCCCATAGTAAAGGTGGGTTATATCCA | 411 |
| SEQ ID NO: 077 | D2_s_292 | TGTAAAACGACGGCCAGTAGTCCATTCATTTAAAACTGGCTTT | |
| SEQ ID NO: 078 | D2_as_292 | CAGGAAACAGCTATGACCTGTTCCCTGTGCTTTCAAATCTTTA | 491 |
| SEQ ID NO: 079 | D2_s_293 | TGTAAAACGACGGCCAGTCCAAAGGAGACAGAAACATCAGAA | |
| SEQ ID NO: 080 | D2_as_293 | CAGGAAACAGCTATGACCGTTAACCAGGCCTCAAATTCTAATT | 578 |
| SEQ ID NO: 081 | D2_s_294 | TGTAAAACGACGGCCAGTCCACAGATTCAGTTCAGATTCCAAA | |
| SEQ ID NO: 082 | D2_as_294 | CAGGAAACAGCTATGACCGGTTATGTCCTACCAGTTCAGGATA | 430 |
| SEQ ID NO: 083 | D2_s_295 | TGTAAAACGACGGCCAGTGCATGTTCATCTCTCCCAGTATTTC | |
| SEQ ID NO: 084 | D2_as_295 | CAGGAAACAGCTATGACCTAGTACAACAGACTAACTCATGTCT | 462 |
| SEQ ID NO: 085 | D2_s_296 | TGTAAAACGACGGCCAGTTCTACAAAACCCCACTGACTACTAT | |
| SEQ ID NO: 086 | D2_as_296 | CAGGAAACAGCTATGACCAATTCACAAAGTATCACTGCAACTC | 426 |
| SEQ ID NO: 087 | D2_s_297 | TGTAAAACGACGGCCAGTTAACCTTTTCGTAACCAGGCATTAT | |
| SEQ ID NO: 088 | D2_as_297 | CAGGAAACAGCTATGACCACACGATTGACACATCCTGAATTTA | 605 |
| SEQ ID NO: 089 | D2_s_298 | TGTAAAACGACGGCCAGTGACTTAATGGGACTGCTAGAATCTG | |
| SEQ ID NO: 090 | D2_as_298 | CAGGAAACAGCTATGACCTCTGATATTACCAATGCAGTATAGGC | 534 |
| SEQ ID NO: 091 | D2_s_299 | TGTAAAACGACGGCCAGTGCTTTCTCTGACTCATAATTCCCAG | |
| SEQ ID NO: 092 | D2_as_299 | CAGGAAACAGCTATGACCCGGCATAAACTGATAATTTGGTGAC | 560 |
| SEQ ID NO: 093 | D2_s_300 | TGTAAAACGACGGCCAGTGCCCTCTTAAGTCTTTCTCAATCTAG | |
| SEQ ID NO: 094 | D2_as_300 | CAGGAAACAGCTATGACCTCTTCAGAGTTATAGAGCCGAGC | 468 |
| SEQ ID NO: 095 | D2_s_301 | TGTAAAACGACGGCCAGTTCTTCATCTCCTACTTCTGAACAGT | |
| SEQ ID NO: 096 | D2_as_301 | CAGGAAACAGCTATGACCGGAAATCTGGCCAAAAGTTTATCG | 407 |
| SEQ ID NO: 097 | D2_s_302 | TGTAAAACGACGGCCAGTAAAAGACTCTTGACTCCCTTTCTCT | |
| SEQ ID NO: 098 | D2_as_302 | CAGGAAACAGCTATGACCCATCTGAGCTATGAATGGAAGAGC | 616 |
| SEQ ID NO: 099 | D2_s_303 | TGTAAAACGACGGCCAGTTCTTCTCATCTCACAGTTTACCCTA | |
| SEQ ID NO: 100 | D2_as_303 | CAGGAAACAGCTATGACCCTACACTGTGTTGCCATGATAAACT | 613 |
| SEQ ID NO: 101 | D2_s_304 | TGTAAAACGACGGCCAGTGATGAGCAAGTTGATATCCAGTCAT | |
| SEQ ID NO: 102 | D2_as_304 | CAGGAAACAGCTATGACCAAAGGGGAAATATGTAAGGGAATGC | 532 |
| SEQ ID NO: 103 | D2_s_305 | TGTAAAACGACGGCCAGTCTCCATCCAAAACTTCTCGAAAAGA | |
| SEQ ID NO: 104 | D2_as_305 | CAGGAAACAGCTATGACCTTTTGTCCCGAAGAATTGGTCATAA | 636 |
| SEQ ID NO: 105 | D2_s_306 | TGTAAAACGACGGCCAGTGAAGTCACTTTTATGCACAATCCAG | |
| SEQ ID NO: 106 | D2_as_306 | CAGGAAACAGCTATGACCAGAACTGCCAATATATTCTGCATGT | 565 |
| SEQ ID NO: 107 | D2_s_307 | TGTAAAACGACGGCCAGTCTGATGAAAACCCAAGAGCCAG | |
| SEQ ID NO: 108 | D2_as_307 | CAGGAAACAGCTATGACCGTCTGATAATGGTTTGTTTGGAAGT | 494 |
| SEQ ID NO: 109 | D2_s_308 | TGTAAAACGACGGCCAGTCCTGGAAGATACAATCAAGAAAACC | |
| SEQ ID NO: 110 | D2_as_308 | CAGGAAACAGCTATGACCCATATACTTTCTGCACCACCTCATA | 489 |
| SEQ ID NO: 111 | D2_s_309 | TGTAAAACGACGGCCAGTGACCTCAAGTGTTAGTTCTGTTGAA | |
| SEQ ID NO: 112 | D2_as_309 | CAGGAAACAGCTATGACCTGTATAGTACCCACCCAAGTTATTT | 418 |
| SEQ ID NO: 113 | D2_s_310 | TGTAAAACGACGGCCAGTCAGCAGGATGACTCTCAATTTTCT | |
| SEQ ID NO: 114 | D2_as_310 | CAGGAAACAGCTATGACCTTCTCCAAACTTTAGTCACGTCTC | 504 |
| SEQ ID NO: 115 | D2_s_311 | TGTAAAACGACGGCCAGTGTAAAATGTGTTAGCCAAGTCCATG | |
| SEQ ID NO: 116 | D2_as_311 | CAGGAAACAGCTATGACCGCACATGTGATTATAAACACAAACA | 423 |
| SEQ ID NO: 117 | D2_s_312 | TGTAAAACGACGGCCAGTGCAACTTTTATCCCAGCCTGAAG | |
| SEQ ID NO: 118 | D2_as_312 | CAGGAAACAGCTATGACCCTGAAGTCTCTGGGTTAGTAAGGAA | 582 |
| SEQ ID NO: 119 | D2_s_313 | TGTAAAACGACGGCCAGTGTATTCCTTGCTCACTTGCTACTAG | |
| SEQ ID NO: 120 | D2_as_313 | CAGGAAACAGCTATGACCAGTGGAGTAAACTTTAAGGATGAGT | 530 |
| SEQ ID NO: 121 | D2_s_314 | TGTAAAACGACGGCCAGTCTCTTTGAGGCATTTGTTTGGAAAA | |
| SEQ ID NO: 122 | D2_as_314 | CAGGAAACAGCTATGACCACTCACATAGAAATAGAAGCCAGAA | 681 |
| SEQ ID NO: 123 | D2_s_315 | TGTAAAACGACGGCCAGTGACTGTTTTCCCCTGTTAATGAGAT | |
| SEQ ID NO: 124 | D2_as_315 | CAGGAAACAGCTATGACCATTGATGACTGCTGATAACCAAAAT | 443 |
| SEQ ID NO: 125 | D2_s_316 | TGTAAAACGACGGCCAGTATATACTTAAGCCTCCATTCCCTCA | |
| SEQ ID NO: 126 | D2_as_316 | CAGGAAACAGCTATGACCTTATCGTTCCTATGGACAGTTGAAG | 527 |
| SEQ ID NO: 127 | D2_s_317 | TGTAAAACGACGGCCAGTTGATTTAGGTTCCTGACACTGATTC | |
| SEQ ID NO: 128 | D2_as_317 | CAGGAAACAGCTATGACCAATCTCAGCAAAGGTAGGTACACTA | 648 |
| SEQ ID NO: 129 | D2_s_318 | TGTAAAACGACGGCCAGTTTGTTTCCAAAATCTTTCCCATCTC | |
| SEQ ID NO: 130 | D2_as_318 | CAGGAAACAGCTATGACCAATCTAGGTTAGTAAGGTCCTCAGC | 542 |
| SEQ ID NO: 131 | D2_s_319 | TGTAAAACGACGGCCAGTTTCAGTACCTCAATTTCTTAGGCAC | |
| SEQ ID NO: 132 | D2_as_319 | CAGGAAACAGCTATGACCGTAGGTTGAGGATCCAGATAGACAT | 687 |
| SEQ ID NO: 133 | D2_s_320 | TGTAAAACGACGGCCAGTGCTTTTCATTTGTGTTATGTAGGGTAC | |
| SEQ ID NO: 134 | D2_as_320 | CAGGAAACAGCTATGACCAAAGTCCATCCATTTACAGGTCCTA | 528 |
| SEQ ID NO: 135 | D2_s_321 | TGTAAAACGACGGCCAGTTAAGAAGTCCACCATATAGCAGTCA | |
| SEQ ID NO: 136 | D2_as_321 | CAGGAAACAGCTATGACCGTTCTCCCTTCTTACCCTTATTTCC | 416 |
| SEQ ID NO: 137 | D2_s_322 | TGTAAAACGACGGCCAGTAGATTCACAGTTCATTTCAGGAAGA | |
| SEQ ID NO: 138 | D2_as_322 | CAGGAAACAGCTATGACCAAAATGAGTGGCAGAGGAATACTAC | 365 |
| SEQ ID NO: 139 | D2_s_323 | TGTAAAACGACGGCCAGTTAGGACTGATAGGGACCAAGATTAC | |
| SEQ ID NO: 140 | D2_as_323 | CAGGAAACAGCTATGACCATATGTTGACTTACAGATTCCCCAC | 539 |
| SEQ ID NO: 141 | D2_s_324 | TGTAAAACGACGGCCAGTAAACCAAAGGAAAGAAATAATCGGA | |
| SEQ ID NO: 142 | D2_as_324 | CAGGAAACAGCTATGACCCCCTCTTATCAAGCTTTCATCACC | 543 |
| SEQ ID NO: 143 | D2_s_325 | TGTAAAACGACGGCCAGTTGATAGGATGTGTCAATGTTCAGTAT | |
| SEQ ID NO: 144 | D2_as_325 | CAGGAAACAGCTATGACCTTTTGAAACAGAGAGGACTTGGC | 535 |
| SEQ ID NO: 145 | D2_s_326 | TGTAAAACGACGGCCAGTGGACAAAACAATTTATAGGAAGCCA | |
| SEQ ID NO: 146 | D2_as_326 | CAGGAAACAGCTATGACCTTAAGAGGAAAAGAAGTTGAGGCAG | 402 |
| SEQ ID NO: 147 | D2_s_327 | TGTAAAACGACGGCCAGTGGAATTCACCCTAAATCTCAACTGA | |
| SEQ ID NO: 148 | D2_as_327 | CAGGAAACAGCTATGACCACTCTTTCTAATCTGCCCAATGATG | 539 |
| SEQ ID NO: 149 | D2_s_328 | TGTAAAACGACGGCCAGTAGATCCCAGTACATATCCTTTACGT | |
| SEQ ID NO: 150 | D2_as_328 | CAGGAAACAGCTATGACCTGTAAAGGAAAATGGCAGGTTACAT | 634 |
| SEQ ID NO: 151 | D2_s_329 | TGTAAAACGACGGCCAGTTCTCACTACCTCTAATCAGCTTCTT | |
| SEQ ID NO: 152 | D2_as_329 | CAGGAAACAGCTATGACCTCCTAGTACTCTCCCAGGATAGC | 552 |
| SEQ ID NO: 153 | D2_s_330 | TGTAAAACGACGGCCAGTCTTTTGAGCGAATGGTTTTATGTGT | |
| SEQ ID NO: 154 | D2_as_330 | CAGGAAACAGCTATGACCTGCATACTTACTGCTTGACTACATC | 534 |
| SEQ ID NO: 155 | D2_s_331 | TGTAAAACGACGGCCAGTCCAATTAAAACTCATGCCCCTTAG | |
| SEQ ID NO: 156 | D2_as_331 | CAGGAAACAGCTATGACCCTTGAATTCTATAGGTTTGTGCTGG | 500 |
| SEQ ID NO: 157 | D2_s_332 | TGTAAAACGACGGCCAGTCTAAGGGACATGACAGAGGTTCT | |
| SEQ ID NO: 158 | D2_as_332 | CAGGAAACAGCTATGACCCTTACGGTGTCCCTAGCATTTTAG | 409 |
| SEQ ID NO: 159 | D2_s_333 | TGTAAAACGACGGCCAGTGTCCTTTACCTATGAAGTGCTAGG | |
| SEQ ID NO: 160 | D2_as_333 | CAGGAAACAGCTATGACCCACCATGCCCTAGTGTCTAATTT | 479 |
| SEQ ID NO: 161 | D2_s_334 | TGTAAAACGACGGCCAGTACGTTTTCACCTGCCATTTAATTG | |
| SEQ ID NO: 162 | D2_as_334 | CAGGAAACAGCTATGACCTGTCTTCTCTCTACAAGGACCTCA | 440 |
| SEQ ID NO: 163 | D2_s_335 | TGTAAAACGACGGCCAGTGAAAAGGAAAGGTAGGTAGGTGTTG | |
| SEQ ID NO: 164 | D2_as_335 | CAGGAAACAGCTATGACCGGACAAGACGTATAAGACTACTCCT | 464 |
| SEQ ID NO: 165 | D2_s_336 | TGTAAAACGACGGCCAGTGTTGATGTACACTGGAGAAAGGG | |
| SEQ ID NO: 166 | D2_as_336 | CAGGAAACAGCTATGACCGTTAAGAAGGCAGGCAATGAGAC | 447 |
| SEQ ID NO: 167 | D2_s_337 | TGTAAAACGACGGCCAGTGAATTTATCTAGGACCCCGTGC | |
| SEQ ID NO: 168 | D2_as_337 | CAGGAAACAGCTATGACCATCTGACAGGAGTTACAAGAAAGGA | 485 |
| SEQ ID NO: 169 | D2_s_338 | TGTAAAACGACGGCCAGTCTCAGCCTCTCATTTCTAAGGTTTA | |
| SEQ ID NO: 170 | D2_as_338 | CAGGAAACAGCTATGACCTTCCTCTCTAGTTACCTTCTTCTCC | 412 |
| SEQ ID NO: 171 | D2_s_339 | TGTAAAACGACGGCCAGTTGTTGTGGATTCCTAGAAATGAAGG | |
| SEQ ID NO: 172 | D2_as_339 | CAGGAAACAGCTATGACCCTACCCTAGCACAGATGGATGC | 462 |
| SEQ ID NO: 173 | D2_s_340 | TGTAAAACGACGGCCAGTCTAGGCAAACACAGTTACTCCTC | |
| SEQ ID NO: 174 | D2_as_340 | CAGGAAACAGCTATGACCACTCTTTACCAGATCCAGTATAGGC | 450 |
| SEQ ID NO: 175 | D2_s_341 | TGTAAAACGACGGCCAGTGAGCCTGAGTTCTCATTTGCCTAT | |
| SEQ ID NO: 176 | D2_as_341 | CAGGAAACAGCTATGACCCATGATTCTTTTATCTCTCCTGGGG | 371 |
| SEQ ID NO: 177 | D2_s_342 | TGTAAAACGACGGCCAGTCTATCCCTGTTTCAACTCTTTCCAG | |
| SEQ ID NO: 178 | D2_as_342 | CAGGAAACAGCTATGACCCCACACATTATTCTACCCTGCTG | 555 |
| SEQ ID NO: 179 | D2_s_343 | TGTAAAACGACGGCCAGTCATTCTATGTGTCCAGTCTTAGGC | |
| SEQ ID NO: 180 | D2_as_343 | CAGGAAACAGCTATGACCGGTGTCAAACTAGAAGATGTGTAGG | 553 |
| SEQ ID NO: 181 | D2_s_344 | TGTAAAACGACGGCCAGTGAGACCTATGATAAGTGAGCTCTCT | |
| SEQ ID NO: 182 | D2_as_344 | CAGGAAACAGCTATGACCTAGCCTCAGTGTTTATATCCCACA | 657 |
| SEQ ID NO: 183 | D2_s_345 | TGTAAAACGACGGCCAGTCTAGAGATACCCATTCTGAACCTCT | |
| SEQ ID NO: 184 | D2_as_345 | CAGGAAACAGCTATGACCGAAAATGCGTTGAAGTTGAGAGT | 551 |
| SEQ ID NO: 185 | D2_s_346 | TGTAAAACGACGGCCAGTGCCTAATTCTTCTAACACTCTGCT | |
| SEQ ID NO: 186 | D2_as_346 | CAGGAAACAGCTATGACCTGGGACACACACTATCATAGACTC | 480 |
| SEQ ID NO: 187 | D2_s_347 | TGTAAAACGACGGCCAGTCCACCTCTATATTCAGTCAAAGGAG | |
| SEQ ID NO: 188 | D2_as_347 | CAGGAAACAGCTATGACCAGGGACATAGACTGGAAGTGGC | 533 |
| SEQ ID NO: 189 | D2_s_348 | TGTAAAACGACGGCCAGTTAAGTTCTGGAAAAGGTCTGATTGC | |
| SEQ ID NO: 190 | D2_as_348 | CAGGAAACAGCTATGACCGGGTGAATCTGAGTGTCTTAGGT | 546 |
| SEQ ID NO: 191 | D2_s_349 | TGTAAAACGACGGCCAGTAGGAAGTAGGTTAGGGAATGGTAAG | |
| SEQ ID NO: 192 | D2_as_349 | CAGGAAACAGCTATGACCCAGTGACATCAGAGAATCAAAGGAG | 355 |
| SEQ ID NO: 193 | D2_s_350 | TGTAAAACGACGGCCAGTAGAACATCTCTCTGGCCTAAACT | |
| SEQ ID NO: 194 | D2_as_350 | CAGGAAACAGCTATGACCGTTTAACCATCTCTTTCCCTGAAGT | 424 |
| SEQ ID NO: 195 | D2_s_351 | TGTAAAACGACGGCCAGTTTACCATCTGACCTCAAACAATGTT | |
| SEQ ID NO: 196 | D2_as_351 | CAGGAAACAGCTATGACCCAATCTATGCCTGCAGTGGAATC | 480 |
| SEQ ID NO: 197 | D2_s_352 | TGTAAAACGACGGCCAGTGCCTTCTTTTATTCATGCGTTGTTT | |
| SEQ ID NO: 198 | D2_as_352 | CAGGAAACAGCTATGACCCCTTCTCTTCTTGTTTCCTGGTAA | 531 |

## Patentansprüche

1. Verfahren zur Detektion von Chromosomen- oder DNA-Bereichen und zum Nachweis von Chromosomenaberrationen, bei dem direkt oder indirekt markierte Nukleinsäurefragmente (DNA-Sonden) für eine chromogene oder fluoreszierende *in-situ*-Hybridisierung (CISH; FISH) von Chromosomen mit vermindertem Hintergrund verwendet werden, wobei die DNA-Sonden hergestellt sind mit einem Verfahren umfassend die Schritte:
a) Auswählen einer Anzahl einmal vorkommender Nukleinsäuresequenzen in einem längerem Genomabschnitt durch Sequenzvergleich;
b) Synthetisieren von *Sense-* und *Antisense*-Primern für eine Polymerasekettenreaktion an der Anzahl der ausgewählten Nukleinsäuresequenzen, wobei die Primer jeweils eine Sequenz komplementär zum Strang oder Gegenstrang der ausgewählten Nukleinsäuresequenz aufweisen sowie mindestens eine einheitliche Oligonukleotidsequenz, die unter stringenten Bedingungen nicht mit einer Sequenz des Genoms hybridisiert;
c) Durchführen von Polymerasekettenreaktionen mit der Anzahl *Sense-* und *Antisense-Primer* am Genomabschnitt und Erhalten von synthetisierten Nukleinsäurefragmenten, die bekannte einmal vorkommende Genomsequenzen enthalten;
d) Durchführen einer zweiten Polymerasekettenreaktion an den synthetisierten Nukleinsäurefragmenten von Schritt c) unter Verwendung einer Anzahl von Primern, die mit der einheitlichen Oligonukleotidsequenz der Primer hybridisieren und Erhalten amplifizierter einmal vorkommender genomischer Nukleinsäurefragmente, welche eingesetzt werden können für die besagte chromogene oder fluoreszierende *in-situ*-Hybridisierung von Chromosomen mit vermindertem Hintergrund.

2. Verfahren nach Anspruch 1, wobei eine Anzahl der nach der Polymerasekettenreaktion in Schritt (c) erhaltenen synthetisierten Nukleinsäurenfragmente vor der zweiten Polymerasekettenreaktion zusammen gegeben werden, bevorzugt vor ihrer Aufreinigung.

3. Verfahren nach Anspruch 1 oder 2, wobei in Schritt (d) eine Markierung oder Aktivierung der Nukleinsäurefragmente erfolgt durch den Einsatz von modifizierten oder markierten Nukleotiden, fluoreszierend oder chromogen markierte Nukleotiden (NTPs) und dNTPs, haptenmarkierten Nukleotiden, chemisch aktiven Nukleotiden, Aminoallyl-Nukleotiden.

4. Verfahren nach Anspruch 1 oder 2, wobei die nach der ersten Polymerasekettenreaktion in Schritt (c) erhaltenen genomischen Nukleinsäurefragmente in Plasmide kloniert werden.

5. Verfahren nach einem der Ansprüche 1 bis 4, wobei nach Schritt d) eine Umsetzung folgt für einen Einbau von Nachweisgruppen in die genomischen Nukleinsäurefragmente, ausgewählt aus Nicktranslation, chemische Umsetzung, immunologische Reaktion.

6. Verfahren nach einem der Ansprüche 1 bis 5, wobei in Schritt (a) die genomischen Nukleinsäurensequenzen so ausgewählt werden, dass Produkte mit ähnlicher Größe erzeugt werden.

7. Verfahren nach einem der Ansprüche 1 bis 6, wobei die in Schritt (a) ausgewählten genomischen Nukleinsäuresequenzen zwischen 100 bis 1000 Basenpaare, besonders bevorzugt 400 bis 600 Basenpaaren besitzen.

8. Verfahren nach einem der Ansprüche 1 bis 5, wobei die in Schritt (a) ausgewählten genomischen Nukleinsäurensequenzen auf dem Genom zueinander benachbart sind.

9. Verfahren nach einem der Ansprüche 1 bis 8, wobei Sonden mit unterschiedlichen Markierungen für die in-situ-Hybridisierung hergestellt werden.

10. Verfahren nach einem der Ansprüche 1 bis 9, die in Schritt (a) ausgewählten genomischen Nukleinsäuresequenzen zu einem Bruchbereich des Chromosoms benachbart sind oder diesen flankieren.

11. Verfahren nach einem der Ansprüche 1 bis 10, wobei die Markierungen der DNA-Sonden so ausgewählt sind, dass sie bei der in-situ-Hybridisierung von Chromosomen ein Fusionssignal oder ein verändertes Fusionssignal erzeugen.

12. Verfahren nach einem der Ansprüche 1 bis 11, wobei die Markierung ausgewählt ist aus der Gruppe der farbgebenden Moleküle, Polymethin-Farbstoffe, Thiazol- und Oxazolfarbstoffe, Hoechst 33342 (2'-(4-Ethoxyphenyl)-5-(4-methyl-1-piperazinyl)-2,5'-bi-1H-benzimidazol-trihydrochlorid), 4',6-Diamidin-2-phenylindol, Alexa 405, Alexa 488, Alexa 594, Alexa 633; Texas Red, Rhodamin; sulfonierte und nicht-sulfonierte Cyanin-Farbstoffe, Cy2, Cy3, Cy5, Cy7; fluoreszierenden Moleküle, Fluorescein, 5,6-Carboxfluorescein, FITC (Fluoresceinisothiocyanat), GFP (grünfluoreszierendes Protein); chemilumineszierende Mokeküle, Acridinium; ; ATTO®-Fluoreszenzfarbstoffe (Atto-Tec, Siegen, DE), PromoFluor®-Farbstoffe (PromoCell GmbH, Heidelberg, DE), MoBiTec®-Farbstoffe (MoBiTec GmbH, Göttingen, DE), DY®-Farbstoffe (DYOMICS GmbH, Jena, DE) Quantum Dots; Haptene, Digoxigenin, Biotin, 2,4-Dinitrophenol, Avidin; Enzyme für eine farbgebende Reaktion, Peroxidase, Meerrettichperoxidase, alkalische Phosphatase.

13. Verwendung von CISH- oder FISH-Sonden in einem Verfahren für eine in-situ-Hybridisierung zum Nachweis einer Chromosomenaberration gemäß einem der vorhergehenden Ansprüche, wobei die CISH- oder FISH-Sonden aus einer Anzahl synthetisierter PCR-Fragmente bestehen und hergestellt sind mit einem Verfahren umfassend die Schritte:
a) Auswählen einer Anzahl einmal vorkommender Nukleinsäuresequenzen in einem längerem Genomabschnitt durch Sequenzvergleich;
b) Synthetisieren von *Sense-* und *Antisense*-Primern für eine Polymerasekettenreaktion an der Anzahl der ausgewählten Nukleinsäuresequenzen, wobei die Primer jeweils eine Sequenz komplementär zum Strang oder Gegenstrang der ausgewählten Nukleinsäuresequenz aufweisen sowie mindestens eine einheitliche Oligonukleotidsequenz, die unter stringenten Bedingungen nicht mit einer Sequenz des Genoms hybridisiert;
c) Durchführen von Polymerasekettenreaktionen mit der Anzahl *Sense-* und *Antisense-Primer* am Genomabschnitt und Erhalten von synthetisierten Nukleinsäurefragmenten, die bekannte einmal vorkommende Genomsequenzen enthalten;
d) Durchführen einer zweiten Polymerasekettenreaktion an den synthetisierten Nukleinsäurefragmenten von Schritt c) unter Verwendung einer Anzahl von Primern, die mit der einheitlichen Oligonukleotidsequenz der Primer hybridisieren und Erhalten amplifizierter einmal vorkommender genomischer Nukleinsäurefragmente, welche eingesetzt werden können für eine chromogene oder fluoreszierende *in-situ*-Hybridisierung von Chromosomen mit vermindertem Hintergrund.

14. Verwendung eines CISH- oder FISH-Sondengemisches oder Testbestecks in einem Verfahren für den Nachweis einer Chromosomenaberration nach einem der Ansprüche 1 bis 12, wobei das CISH- oder FISH-Sondengemisch oder Testbesteck mehrere unterschiedlich markierte CISH- oder FISH-Sonden enthält, die hergestellt sind mit einem Verfahren umfassend die Schritte:
a) Auswählen einer Anzahl einmal vorkommender Nukleinsäuresequenzen in einem längerem Genomabschnitt durch Sequenzvergleich;
b) Synthetisieren von *Sense-* und *Antisense*-Primern für eine Polymerasekettenreaktion an der Anzahl der ausgewählten Nukleinsäuresequenzen, wobei die Primer jeweils eine Sequenz komplementär zum Strang oder Gegenstrang der ausgewählten Nukleinsäuresequenz aufweisen sowie mindestens eine einheitliche Oligonukleotidsequenz, die unter stringenten Bedingungen nicht mit einer Sequenz des Genoms hybridisiert;
c) Durchführen von Polymerasekettenreaktionen mit der Anzahl *Sense-* und *Antisense-Primer* am Genomabschnitt und Erhalten von synthetisierten Nukleinsäurefragmenten, die bekannte einmal vorkommende Genomsequenzen enthalten;
d) Durchführen einer zweiten Polymerasekettenreaktion an den synthetisierten Nukleinsäurefragmenten von Schritt c) unter Verwendung einer Anzahl von Primern, die mit der einheitlichen Oligonukleotidsequenz der Primer hybridisieren und Erhalten amplifizierter einmal vorkommender genomischer Nukleinsäurefragmente, welche eingesetzt werden können für eine chromogene oder fluoreszierende *in-situ*-Hybridisierung von Chromosomen mit vermindertem Hintergrund.

## Claims

1. Method for the detection of chromosome or DNA regions and for the detection of chromosome aberrations, in which directly or indirectly labeled nucleic acid fragments (DNA probes) are used for a chromogenic or fluorescence *in-situ* hybridization (CISH; FISH) of chromosomes with a reduced background, wherein the DNA probes are produced with a method comprising the steps:
a) Selecting a number of nucleic acid sequences which occur once in a longer genome section by comparing sequences;
b) Synthesizing *sense* and *antisense* primers for a polymerase chain reaction on the number of the selected nucleic acid sequences, wherein each primer has a sequence which is complementary to the strand or complementary strand of the selected nucleic acid sequence, and also has at least one uniform oligonucleotide sequence which does not hybridize with a sequence of the genome under stringent conditions;
c) Performing polymerase chain reactions with the number of *sense* and *antisense* primers on the genome section and obtaining synthesized nucleic acid fragments which contain known genome sequences which occur once;
d) Performing a second polymerase chain reaction on the synthesized nucleic acid fragments of step c) using a number of primers which hybridize with the uniform oligonucleotide sequence of the primers and obtaining amplified genomic nucleic acid fragments which occur once and which can be used for the aforementioned chromogenic or fluorescence *in-situ* hybridization of chromosomes with a reduced background.

2. The method according to Claim 1, wherein a number of the synthesized nucleic acid fragments obtained after the polymerase chain reaction in step (c) are combined before the second polymerase chain reaction, preferably before their purification.

3. The method according to Claim 1 or 2, wherein in step (d) the nucleic acid fragments are labeled or activated by the use of modified or labeled nucleotides, fluorescent or chromo-genically labeled nucleotides (NTPs) and dNTPs, hapten-labeled nucleotides, chemically active nucleotides, aminoallyl nucleotides.

4. The method according to Claim 1 or 2, wherein the genomic nucleic acid fragments obtained after the first polymerase chain reaction in step (c) are cloned into plasmids.

5. The method according to one of the Claims 1 to 4, wherein after step d) a reaction follows to insert reporter groups into the genomic nucleic acid fragments, selected from nick translation, chemical reaction, immunological reaction.

6. The method according to one of the Claims 1 to 5, wherein in step (a) the genomic nucleic acid sequences are selected such that products of similar size are produced.

7. The method according to one of the Claims 1 to 6, wherein the genomic nucleic acid sequences selected in step (a) have between 100 and 1,000 base pairs, most preferably between 400 and 600 base pairs.

8. The method according to one of the Claims 1 to 5, wherein the genomic nucleic acid sequences selected in step (a) are adjacent to each other on the genome.

9. The method according to one of the Claims 1 to 8, wherein probes with different labels are produced for the in-situ hybridization.

10. The method according to one of the Claims 1 to 9, wherein the genomic nucleic acid sequences selected in step (a) are adjacent to a breakpoint region of the chromosome or flank this region.

11. The method according to one of the Claims 1 to 10, wherein the labels of the DNA probes are selected such that they create a fusion signal or a modified fusion signal in the in-situ hybridization of chromosomes.

12. The method according to one of the Claims 1 to 11, wherein the label is selected from the group of chromogenic molecules, polymethine dyes, thiazole and oxazole dyes, Hoechst 33342 (2'-(4-ethoxyphenyl)-5-(4-methyl-1-piperazinyl)-2,5'-bi-1H-benzimidazole trihydrochloride), 4',6-diamidin-2-phenylindole, Alexa 405, Alexa 488, Alexa 594, Alexa 633; Texas Red, rhodamine; sulfonated and non-sulfonated cyanine dyes, Cy2, Cy3, Cy5, Cy7; fluorescent molecules, fluorescein, 5,6-carbofluorescin, FITC (fluorescein isothiocyanate), GFP (green fluorescent protein); chemiluminescent molecules, acridinium; ATTO®-fluorescent dyes (Atto-Tec, Siegen, DE), PromoFluor® dyes (PromoCell GmbH, Heidelberg, DE), MoBiTec® dyes (MoBiTec GmbH, Goettingen, DE), DY® dyes (DYOMICS GmbH, Jena, DE) Quantum Dots; haptens, digoxigenin, biotin, 2,4-dinitrophenol, avidin; enzymes for a chromogenic reaction, peroxidase, horseradish peroxidase, alkaline phosphatase.

13. Use of CISH or FISH probes in a method for in situ hybridization for detecting a chromosomal aberration according to any of the preceding claims, wherein the CISH or FISH probes consist of a number of synthesized PCR fragments and are produced by a method comprising the following steps:
(a) Selecting a number of nucleic acid sequences which occur once in a longer genome section by comparing sequences;
b) Synthesizing *sense* and *antisense* primers for a polymerase chain reaction on the number of the selected nucleic acid sequences, wherein each primer has a sequence which is complementary to the strand or complementary strand of the selected nucleic acid sequence, and also has at least one uniform oligonucleotide sequence which does not hybridize with a sequence of the genome under stringent conditions;
c) Performing polymerase chain reactions with the number of sense and antisense primers on the genome segment and obtaining synthesized nucleic acid fragments which contain known genome sequences which occur once;
d) Performing a second polymerase chain reaction on the synthesized nucleic acid fragments of step c) using a number of primers which hybridize to the uniform oligonucleotide sequence of the primers and obtaining amplified genomic nucleic acid fragments which occur once and which can be used for the aforementioned chromogenic or fluorescence *in-situ* hybridization of chromosomes with a reduced background.

14. Use of a CISH or FISH probe mixture or test kit in a method for detecting a chromosomal aberration according to any one of claims 1 to 12, wherein the CISH or FISH probe mixture or test kit comprises a plurality of differently labeled CISH or FISH probes prepared by a method comprising the steps of:
(a) Selecting a number of nucleic acid sequences which occur once in a longer genome section by comparing sequences;
b) Synthesizing *sense* and *antisense* primers for a polymerase chain reaction on the number of the selected nucleic acid sequences, wherein each primer has a sequence which is complementary to the strand or complementary strand of the selected nucleic acid sequence, and also has at least one uniform oligonucleotide sequence which does not hybridize with a sequence of the genome under stringent conditions;
c) Performing polymerase chain reactions with the number of sense and antisense primers on the genome segment and obtaining synthesized nucleic acid fragments which contain known genome sequences which occur once;
d) Performing a second polymerase chain reaction on the synthesized nucleic acid fragments of step c) using a number of primers which hybridize to the uniform oligonucleotide sequence of the primers and obtaining amplified genomic nucleic acid fragments which occur once and which can be used for the aforementioned chromogenic or fluorescence in-situ hybridization of chromosomes with a reduced background.

## Revendications

1. Méthode utilisée pour détecter des régions chromosomiques ou d'ADN et pour apporter la preuve d'aberrations chromosomiques, étant entendu que les fragments d'acide nucléique directement ou indirectement marqués (sondes à ADN) sont utilisés pour l'hybridation chromogène (CISH) ou fluorescente (FISH) in situ de chromosomes avec arrière-plan réduit, les sondes à ADN étant fabriquées lors d'un procédé comprenant les étapes suivantes :
a) Sélection d'un certain nombre de séquences d'acides nucléiques à occurrence unique dans un segment génomique plus long par comparaison de séquences ;
b) Synthèse d'amorces sens et antisens pour une réaction en chaîne par polymérase au niveau du nombre de séquences d'acides nucléiques sélectionnées, étant entendu que chacune des amorces présente une séquence complémentaire du brin sens ou antisens de la séquence d'acides nucléiques sélectionnée ainsi qu'au moins une séquence uniforme d'oligonucléotides qui, dans des conditions très strictes, n'hybride pas avec une séquence du génome ;
c) Exécution de réactions en chaîne par polymérase avec le nombre d'amorces sens et antisens sur le segment génomique et obtention de fragments d'acide nucléique synthétisés qui contiennent des séquences génomiques à occurrence unique connues ;
d) Exécution d'une seconde réaction en chaîne par polymérase au niveau des fragments d'acide nucléique synthétisés selon l'étape c), en utilisant un nombre d'amorces qui hybrident avec la séquence d'oligonucléotides uniforme des amorces, et obtention de fragments d'acide nucléique génomiques amplifiés à occurrence unique, lesquels peuvent être employés pour ladite hybridation chromogène ou fluorescente in situ de chromosomes avec arrière-plan réduit.

2. Méthode selon la revendication 1, étant entendu qu'un certain nombre des fragments d'acide nucléique synthétisés obtenus après la réaction en chaîne par polymérase selon l'étape (c) sont regroupés avant la seconde réaction en chaîne par polymérase, de préférence avant leur purification.

3. Méthode selon la revendication 1 ou 2, étant entendu que, lors de l'étape (d), un marquage ou une activation des fragments d'acide nucléique a lieu en utilisant des nucléotides modifiés ou marqués, des nucléotides marqués par fluorescence ou chromogènes (NTPs) et des dNTPs, des nucléotides marqués à l'aide d'haptènes, des nucléotides chimiquement actifs, des nucléotides aminoallyl.

4. Méthode selon la revendication 1 ou 2, étant entendu que les fragments d'acide nucléique génomiques obtenus après la première réaction en chaîne par polymérase selon l'étape (c) sont clonés dans des plasmides.

5. Méthode selon l'une des revendications 1 à 4, étant entendu qu'après l'étape d), une mise en œuvre a lieu pour l'incorporation de groupes de preuve dans les fragments d'acide nucléique génomiques, sélectionnés par translation de coupure (Nick translation), réaction chimique, réaction immunologique.

6. Méthode selon l'une des revendications 1 à 5, étant entendu que, lors de l'étape (a), les séquences d'acides nucléiques génomiques sont sélectionnées de manière à générer des produits de tailles similaires.

7. Méthode selon l'une des revendications 1 à 6, étant entendu que les séquences d'acides nucléiques génomiques sélectionnées lors de l'étape (a) possèdent entre 100 et 1 000 paires de bases, de préférence 400 à 600 paires de bases.

8. Méthode selon l'une des revendications 1 à 5, étant entendu que les séquences d'acides nucléiques génomiques sélectionnées lors de l'étape (a) sont voisines les unes des autres sur le génome.

9. Méthode selon l'une des revendications 1 à 8, étant entendu que des sondes avec différents marquages sont fabriquées pour l'hybridation in situ.

10. Méthode selon l'une des revendications 1 à 9, étant entendu que les séquences d'acides nucléiques génomiques sélectionnées lors de l'étape (a) sont voisines d'un fragment du chromosome ou le flanquent.

11. Méthode selon l'une des revendications 1 à 10, étant entendu que les marquages des sondes à ADN sont sélectionnés de manière à générer lors de l'hybridation in situ de chromosomes un signal de fusion ou un signal de fusion modifié.

12. Méthode selon l'une des revendications 1 à 11, étant entendu que le marquage est sélectionné à partir du groupe des molécules colorantes, colorants polyméthines, colorants thiazole et oxazole, Hoechst 33342 (2'-(4-éthoxyphényl)-5-(4-méthyl-1-pipérazinyl)-2,5'-bi-1H-benzimida-zole- trihydrochloride), 4',6-diamidino-2-phénylindol, Alexa 405, Alexa 488, Alexa 594, Alexa 633 ; Texas Red, Rhodamine ; colorants cyanines sulfonés et non sulfonés, Cy2, Cy3, Cy5, Cy7; molécules fluorescentes, fluorescéine, 5,6-carboxfluorescéine, FITC (IsoThioCyanate de Fluorescéine), GFP (protéine fluorescente verte) ; molécules chimiluminescentes, acridinium ; colorants fluorescents ATTC® (Atto-Tec, Siegen, DE), colorants PromoFluor® (PromoCell GmbH, Heidelberg, DE), colorants MoBiTec® (MoBiTec GmbH, Göttingen, DE), colorants DY® (DYOMICS GmbH, Iéna, DE) Quantum Dots ; haptènes, digoxigénine, biotine, 2,4-dinitrophénol, avidine ; enzymes pour une réaction colorante, peroxydase, peroxydase de raifort, phosphatases alcalines.

13. Utilisation de sondes CISH ou FISH dans une méthode pour hybridation in situ afin d'apporter la preuve d'une aberration chromosomique conformément à l'une des revendications précédentes, étant entendu que les sondes CISH ou FISH sont composées d'un certain nombre de fragments PCR synthétisés et qu'elles sont fabriquées lors d'un procédé comprenant les étapes suivantes :
a) Sélection d'un certain nombre de séquences d'acides nucléiques à occurrence unique dans un segment génomique plus long par comparaison de séquences ;
b) Synthèse d'amorces sens et antisens pour une réaction en chaîne par polymérase au niveau du nombre de séquences d'acides nucléiques sélectionnées, étant entendu que chacune des amorces présente une séquence complémentaire du brin sens ou antisens de la séquence d'acides nucléiques sélectionnée ainsi qu'au moins une séquence uniforme d'oligonucléotides qui, dans des conditions très strictes, n'hybride pas avec une séquence du génome ;
c) Exécution de réactions en chaîne par polymérase avec le nombre d'amorces sens et antisens sur le segment génomique et obtention de fragments d'acide nucléique synthétisés qui contiennent des séquences génomiques à occurrence unique connues ;
d) Exécution d'une seconde réaction en chaîne par polymérase au niveau des fragments d'acide nucléique synthétisés selon l'étape c), en utilisant un nombre d'amorces qui hybrident avec la séquence d'oligonucléotides uniforme des amorces, et obtention de fragments d'acide nucléique génomiques amplifiés à occurrence unique, lesquels peuvent être employés pour ladite hybridation chromogène ou fluorescente in situ de chromosomes avec arrière-plan réduit.

14. Utilisation d'une sonde CISH ou FISH ou d'un kit d'analyse dans une méthode visant à prouver une aberration chromosomique selon l'une des revendications 1 à 12, étant entendu que le mélange de sondes CISH ou FISH ou le kit d'analyse contient plusieurs sondes CISH ou FISH marquées différemment qui sont fabriquées lors d'un procédé comprenant les étapes suivantes :
a) Sélection d'un certain nombre de séquences d'acides nucléiques à occurrence unique dans un segment génomique plus long par comparaison de séquences ;
b) Synthèse d'amorces sens et antisens pour une réaction en chaîne par polymérase au niveau du nombre de séquences d'acides nucléiques sélectionnées, étant entendu que chacune des amorces présente une séquence complémentaire du brin sens ou antisens de la séquence d'acides nucléiques sélectionnée ainsi qu'au moins une séquence uniforme d'oligonucléotides qui, dans des conditions très strictes, n'hybride pas avec une séquence du génome ;
c) Exécution de réactions en chaîne par polymérase avec le nombre d'amorces sens et antisens sur le segment génomique et obtention de fragments d'acide nucléique synthétisés qui contiennent des séquences génomiques à occurrence unique connues ;
d) Exécution d'une seconde réaction en chaîne par polymérase au niveau des fragments d'acide nucléique synthétisés selon l'étape c), en utilisant un nombre d'amorces qui hybrident avec la séquence d'oligonucléotides uniforme des amorces, et obtention de fragments d'acide nucléique génomiques amplifiés à occurrence unique, lesquels peuvent être employés pour une hybridation chromogène ou fluorescente in situ de chromosomes avec arrière-plan réduit.
